# EUROPÄISCHE PATENTSCHRIFT

(11) **EP 4 027 954 B1**
(45) Veröffentlichungstag und Bekanntmachung des Hinweises auf die Patenterteilung: **11.10.2023**
(21) Anmeldenummer: 20764653.0
(22) Anmeldetag: 31.08.2020
(51) Int. Cl.: A61F 2/64, A61F 2/68, A61F 2/60, A61F 2/74

(54) **HYDRAULIKAKTUATOR FÜR ORTHESEN ODER PROTHESEN UND ORTHOPÄDIETECHNISCHE EINRICHTUNG**
HYDRAULIC ACTUATOR FOR ORTHOSES OR PROSTHESES AND ORTHOPEDIC EQUIPMENT
ACTIONNEUR HYDRAULIQUE POUR ORTHÈSES OU PROTHÈSES ET DISPOSITIF TECHNIQUE ORTHOPÉDIQUE

(30) Priorität: 12.09.2019 DE 102019124545
(43) Veröffentlichungstag der Anmeldung: 20.07.2022
(73) Patentinhaber: Otto Bock Healthcare Products GmbH, 1110 Wien (AT)
(72) Erfinder: EDER, Florian, 1040 Wien (AT)
(74) Vertreter: Gramm, Lins & Partner Patent- und Rechtsanwälte PartGmbB
(86) Internationale Anmeldenummer: PCT/EP2020/074220
(87) Internationale Veröffentlichungsnummer: WO 2021/047934

(56) Entgegenhaltungen:
- WO-A1-2010/005473
- WO-A1-2018/161023
- GB-A- 2 566 310
- US-A1- 2015 202 057
- US-A1- 2015 320 573
- US-A1- 2018 098 864
- US-B2- 10 406 001

## Beschreibung

Die Erfindung betrifft einen Hydraulikaktuator für Orthesen oder Prothesen mit einem Stellelement mit einem Gehäuse, in dem ein Zylinder angeordnet ist, dem ein Kolben beweglich gelagert ist und den Zylinder in eine Flexionskammer und eine Extensionskammer unterteilt, wobei zwischen der Flexionskammer und der Extensionskammer eine strömungstechnische Verbindung angeordnet ist, in der je ein Stellventil zur Beeinflussung der Extensionsbewegung bzw. Flexionsbewegung angeordnet ist, wobei eine motorisch angetriebene Pumpe in der strömungstechnischen Verbindung angeordnet ist. Die Erfindung betrifft ebenfalls eine orthopädietechnische Einrichtung, besondere eine Orthese oder Prothese mit einem solchen Hydraulikaktuator.

Orthesen oder Prothesen weisen häufig Gelenkeinrichtungen auf, die ein Oberteil und ein Unterteil aufweisen, die schwenkbar um zumindest eine Schwenkachse aneinander gelagert sind. Die Gelenke sind bei Orthesen in der Regel neben den natürlichen Gelenken angeordnet, beispielsweise neben dem Knöchelgelenk, dem Kniegelenk und/oder dem Hüftgelenk. Ebenso können Orthesen der oberen Extremität oder am Rumpf mit entsprechenden Gelenkeinrichtungen ausgestattet sein. Prothesen ersetzen nicht vorhandene oder nicht mehr vorhandene Gliedmaßen. Die in Prothesen eingesetzten Gelenke ersetzen die jeweiligen natürlichen Gelenke. Um die Bewegung des Oberteils relativ zu dem Unterteil beeinflussen zu können, sind aus dem Stand der Technik rein passive Widerstandseinrichtungen und/oder Dämpfer vorgesehen. Das Widerstandsverhalten dieser Einrichtungen kann fest eingestellt sein, alternativ sind Sensoren an der Prothese oder Orthese oder an der jeweiligen Gliedmaße angeordnet und ermitteln Sensorwerte, auf deren Grundlage die Widerstandseinrichtung oder der Dämpfer angesteuert wird. Bei einem Hydraulikdämpfer werden Ventile geöffnet oder geschlossen oder Strömungsquerschnitte verändert, um Strömungswiderstände zu verändern. Bei anderen Widerstandseinrichtungen können magnetorheologische Eigenschaften verändert, Bremsen aktiviert oder Motoren im Generatorbetrieb geschaltet werden. Die Veränderung der Widerstände kann für die Extensionsbewegung und/oder die Flexionsbewegung erfolgen. Es handelt sich dabei dann um ein sogenanntes mechatronisches System.

Die Veränderung der Widerstände durch den Dämpfer oder die Widerstandseinrichtung kann um einen Antrieb ergänzt werden, sodass aus einer reinen passiven Widerstandseinrichtung ein Aktuator wird, der neben der Bereitstellung unterschiedlicher Widerstände oder Dämpfungseigenschaften wahlweise eine Extension und/oder Flexion bewirkt oder zumindest unterstützt. Hierzu sind in der Regel hydraulische Systeme vorgesehen, da rein pneumatische Systeme nicht mit der notwendigen Präzision betrieben werden können, da das in einem Pneumatiksystem verwendete Fluid kompressibel ist.

Aus der WO 2010/005 473 A1 ist eine orthopädietechnische Einrichtung in Gestalt einer Prothese oder Orthese und ein Verfahren zu deren Steuerung bekannt, bei der eine motorisch betriebene Pumpe Hydraulikfluid in eine Flexionskammer oder in eine Extensionskammer pumpt. Die Pumpe kann in einen passiven Betrieb geschaltet werden, bei dem der Motor nicht angetrieben wird, so dass ein hydraulischer Dämpfer entsteht. Der hydraulische Widerstand wird dann über Stellventile, Rückschlagventile und Schaltventile eingestellt. Nachteilig an einer solchen Ausgestaltung sind die benötigten aktiven Ventile und der Umstand, dass ein Schaltventil geöffnet werden muss, bevor die Pumpe eine aktive Bewegung einleiten oder unterstützen kann. Weiterhin ist nachteilig, dass kein Niederdruckbereich vorhanden ist, der eine Pumpenleckage auffangen könnte.

Die US 2018/098864 A1 betrifft eine Aktuator-Dämpfer-Einheit zum Einsatz in orthetischen oder prothetischen Vorrichtungen mit einem Gehäuse, in dem ein Zylinder ausgebildet ist, in dem ein erster Kolben verlagerbar gelagert und mit einer Kolbenstange gekoppelt ist, die mit einem ersten Ende an dem ersten Kolben angeordnet und mit einem zweiten Ende mit der orthetischen oder prothetischen Vorrichtung koppelbar ist. Der erste Kolben trennt zwei Fluidkammern in dem Zylinder voneinander, wobei zumindest ein weiterer Kolben mit dem ersten Kolben zur Ausbildung zumindest einer weiteren, volumenveränderbaren Fluidkammer gekoppelt ist. Stellventile und eine Pumpe mit einem Rückschlagventil sind in der strömungstechnischen Verbindung zwischen den Fluidkammern angeordnet.

Aufgabe der vorliegenden Erfindung ist es, einen Hydraulikaktuator und eine orthopädietechnische Einrichtung bereitzustellen, mit denen ein störungsfreier Übergang von einem passiven zu einem aktiven Betrieb bei einem minimalen Stellaufwand möglich ist.

Diese Aufgabe wird durch einen Hydraulikaktuator mit den Merkmalen des Hauptanspruches und eine orthopädietechnische Einrichtung mit den Merkmalen des nebengeordneten Anspruchs gelöst. Vorteilhafte Ausgestaltungen und Weiterbildungen der Erfindung sind in den Unteransprüchen, der Beschreibung und den Figuren offenbart.

Der Hydraulikaktuator für Orthesen oder Prothesen mit einem Stellelement mit einem Gehäuse, in dem ein Zylinder angeordnet ist, in dem ein Kolben beweglich gelagert ist und den Zylinder in eine Flexionskammer und eine Extensionskammer unterteilt, wobei zwischen der Flexionskammer und der Extensionskammer eine strömungstechnische Verbindung angeordnet ist, in der je ein Stellventil zur Beeinflussung der Extensionsbewegung bzw. Flexionsbewegung angeordnet ist und wobei eine motorisch angetriebene Pumpe in der strömungstechnischen Verbindung angeordnet ist, sieht vor, dass saugseitig das Hydraulikfluid von der einen Kammer durch zumindest ein Stellventil in einer Anschlussleitung zu der Pumpe geleitet wird und druckseitig ein Rückschlagventil in einer Anschlussleitung von der Pumpe zu der anderen Kammer angeordnet ist und das Rückschlagventil ein Rückströmen des gepumpten Mediums aus dieser Kammer zu der Pumpe entgegen der Förderrichtung der Pumpe sperrt, wobei die druckseitige Anschlussleitung, mit der die Pumpe an die strömungstechnische Verbindung angeschlossen ist, zwischen zwei gleichgerichtet wirkenden, in Reihe angeordneten Rückschlagventilen mündet. Mit einem solchen Hydraulikaktuator ist es möglich, einen rein passiven Betrieb, bei der Hydraulikfluid durch ein entsprechendes Stellventil und gegebenenfalls ein Rückschlagventil durch die Verbindungsleitung von der Extensionskammer in die Flexionskammer bzw. umgekehrt von der Flexionskammer in Extensionskammer geleitet wird, einfach durch Zuschalten der Pumpe in einen aktiven Betrieb umzuschalten, ohne dass ein weiteres Ventil aktiv geschaltet werden müsste. Der Aktuator wird durch Inbetriebnahme der Pumpe und Einspeisen des Hydraulikfluids unter Druck in Richtung auf die entsprechende Kammer zu einem aktiven Aktuator, indem die Pumpe Druckfluid gegen das Rückschlagventil fördert. Bei entsprechender Druckdifferenz, wenn der Druck von der Pumpe größer als der dagegen stehende Druck ist, wird das Rückschlagventil geöffnet und die Pumpe das Hydrauliksystem eingekoppelt. Dieses Einkoppeln erfolgt ohne aktives Schalten der Rückschlagventile, wodurch eine Synchronisation des Anfahrens einer Pumpe und des Öffnens eines Schaltventils überflüssig wird. Dies führt zu einem sanften Einkoppeln der Pumpe in das hydraulische System des Aktuators, ohne dass ein zusätzlicher Stellaufwand der bestehenden Hydraulikventile, beispielsweise der Stellventile, zur Beeinflussung der Extensionsdämpfung und/oder Flexionsdämpfung erforderlich wäre. Darüber hinaus ist es möglich, dass bei einem Übergang von einem passiven Betriebsmodus zu einem aktiven Betriebsmodus die Pumpe voreilend hochlaufen kann, beispielsweise um eine bereits eingeleitete Bewegung zu dämpfen und dieser entgegenzuwirken. So kann einer Flexion durch Anfahren der Pumpe in Extensionsrichtung entgegengewirkt werden, beispielsweise auch in Verbindung mit einer Veränderung des Flexionswiderstandes durch Veränderung der Stellung eines Stellventils. Befindet sich das Ventil zur Beeinflussung der Flexionsbewegung in einem geschlossenen Zustand, kann bereits die Extensionsbewegung und Bewegungsumkehr am Gelenk eingeleitet werden. Eine Bewegungsumkehr am Gelenk ergibt sich dann selbsttätig, sobald der Pumpendruck den Druck an dem Kolben innerhalb des Zylinders übersteigt. Die jeweilige druckseitige Anschlussleitung, mit der die Pumpe an die strömungstechnische Verbindung angeschlossen ist, mündet zwischen zwei gleichgerichtet wirkenden, in Reihe angeordneten Rückschlagventilen, wodurch es möglich ist, neben einer rein passiven Betriebsweise des Hydraulikaktuators den aktiven Betrieb einfach mit geringem Schaltungsaufwand, nämlich durch Aktivierung der Pumpe, zu wechseln

Besonders vorteilhaft ist die Ausgestaltung des Hydraulikaktuators, wenn zwischen der Pumpe und der von der Pumpe mit Druck beaufschlagten Kammer nur ein Rückschlagventil angeordnet ist und das Hydraulikfluid von der Pumpe nur durch ein entsprechend gerichtetes Rückschlagventil und die jeweilige Anschlussleitung zu der gewünschten Kammer geleitet wird. Weitere Stellventile sind in der druckseitigen Anschlussleitung von der Pumpe zu der jeweiligen Kammer nicht mehr vorgesehen, wodurch sich ein vereinfachter Aufbau des Hydraulikaktuators und eine Verringerung des Steuerungsaufwandes ergeben.

Eine Variante der Erfindung sieht vor, dass ein Rückschlagventil eine Verbindung zwischen der druckseitige Anschlussleitung und einer saugseitigen Anschlussleitung sperrt. Insbesondere bei einem Aktuator, bei dem nur in einer Bewegungsrichtung pumpengetrieben eine Bewegung unterstützt werden soll, ist eine solche Anordnung platzsparend und materialsparend. Damit ist es möglich, statt wie bei einer Leitungsführung, bei der die druckseitige Anschlussleitung zwischen zwei gleichgerichtet Rückschlagventilen angeordnet ist, eine saugseitige Verbindungsleitung bzw. eine Niederdruck-Verbindungsleitung zwischen der Extensionskammer und der Flexionskammer durch ein parallel geschaltetes Rückschlagventil zu sperren. Die beiden Rückschlagventile sind gleichgerichtet orientiert, sodass Hydraulikfluid, das von der Pumpe strömt, durch das pumpenseitige Rückschlagventil durchtritt, jedoch nicht das Rückschlagventil öffnen kann, das die Hochdruckseite von der Niederdruckseite trennt. Durch diese Schaltungsanordnung ist es weiterhin möglich, den Aktuator passiv in beiden Richtungen zu betreiben und gleichzeitig eine aktive Betriebsweise in nur einer Richtung zu verwirklichen. Hiermit kann ein Rückschlagventil im Vergleich zu einer in beiden Betriebsrichtungen aktiven und passiven Betriebsweise eingespart werden.

In der strömungstechnischen Verbindung zwischen der Flexionskammer und der Extensionskammer kann eine Zentralleitung ausgebildet sein, in die Ausströmleitungen von der Flexionskammer und der Extensionskammer münden. Von der Zentralleitung führen Zuführleitungen zu saugseitigen Einlässen oder zu zumindest einem saugseitigen Einlass der Pumpe. Sofern der Aktuator in beiden Richtungen aktiv ausgestaltet ist und sowohl die Extensionsbewegung als auch die Flexionsbewegung aktiv unterstützen kann, können mehrere Einlässe vorhanden sein. Alternativ kann ein Auslass in der einen Förderrichtung den Einlass in der anderen Förderrichtung darstellen. Das hydraulische Schaltbild ist dann symmetrisch aufgebaut, wodurch es möglich ist, über die Drehrichtung des Pumpenmotors die Förderrichtung zu wechseln und damit auch die Bewegungsrichtung des Aktuators bzw. des Gelenkes vorzugeben. Voraussetzung hierfür ist, dass die Pumpe in beide Richtungen fördern kann, was beispielsweise bei einer Innenzahnradpumpe, einer Außenzahnradpumpe oder einer Zahnringpumpe der Fall ist. Alle anderen Pumpentypen, die in beiden Richtungen fördern können und die Förderrichtung durch die Drehrichtung bedingt ist, können ebenfalls eingesetzt werden. Die Ausströmleitungen führen bevorzugt durch das jeweilige Stellventil in die Zentralleitung. Je nach Strömungsrichtung kann eine Ausströmleitung gleichzeitig eine druckseitige Anschlussleitung ausbilden.

Eine Weiterbildung der Erfindung sieht vor, dass die Pumpe zwei Druckanschlüsse aufweist, die über je eine Anschlussleitung an die strömungstechnische Verbindung angeschlossen sind. Beide Anschlussleitungen münden zwischen zwei gleichgerichtet wirkenden, in Reihe angeordneten Rückschlagventilen, die als Rückschlagventilpaare ausgebildet sind, wobei die Rückschlagventilpaare entgegengesetzt gerichtete Durchflussrichtungen aufweisen. Mit diesen symmetrischen Schaltungsaufbau ist es möglich, einen Hydraulikaktuator mit nur zwei Stellventilen und vier passiven Rückschlagventilen bereitzustellen, der sowohl in Flexionsrichtung als auch in Extensionsrichtung passiv und aktiv betrieben werden kann. Sobald die Pumpe nicht aktiv ist, also nicht motorisch angetrieben wird, erfolgt automatisch eine passive Schaltung des Hydrauliksystems mit einer Beeinflussung der jeweiligen hydraulischen Widerstände nur über die beiden Stellventile. Eine Leckage und einen Druckverlust in der Pumpe wird durch die besondere Anordnung der Rückschlagventile ausgeschlossen, wodurch die Dichtheitsanforderungen an die Pumpe moderat sind. Die Dichtheit des Hydraulikzylinders wird über die besondere Verschaltung der Rückschlagventile sichergestellt, sodass auch bei einer Sperrung des Hydraulikzylinders durch geschlossene Ventile kein Absinken des Kolbens aufgrund einer Leckage durch die Pumpe erfolgt.

Eine Weiterbildung der Erfindung sieht vor, dass die Zentralleitung zu Rückführleitungen zu der Extensionskammer und der Extensionskammer führt und in der jeweiligen Rückführleitung ein Rückschlagventil angeordnet ist, das ein Rückströmen in die Zentralleitung verhindert. Die Rückführleitungen zweigen von der Zentralleitung ab, wobei bei einem aktiven Betrieb nur in einer Bewegungsrichtung ein Abzweig zum saugseitigen Einlass der Pumpe führt. Bei einer Ausgestaltung für einen aktiven Betrieb in beiden Bewegungsrichtungen ist jeweils ein Abzweig zu dem jeweiligen saugseitigen Einlass vorgesehen, wobei die beiden Abzweige jenseits zweier entgegengesetzt gerichteten Rückschlagventilen angeordnet sind. Somit führt eine Anschlussleitung zu einer Rückführleitung zu der Extensionskammer bzw. der Flexionskammer.

Eine Weiterbildung der Erfindung sieht vor, dass ein Ausgleichsvolumen an der strömungstechnischen Verbindung angeschlossen ist, insbesondere an einer Zentralleitung angeschlossen ist, die zwischen zwei Stellventilen ein Einspeisen von Flüssigkeit in den Strömungskreislauf ermöglicht. Ein Ausgleichsvolumen ist insbesondere dann vorteilhaft, wenn nur eine Kolbenstange aus einem Hydraulikzylinder herausragt, sodass der Hydraulikaktuator mit seinem einen Ende über das Gehäuse und mit einem anderen Ende über eine Kolbenstange an der Orthese oder Prothese oder der orthopädietechnischen Einrichtung allgemein befestigt wird. Das Ausgleichsvolumen kompensiert die Volumenunterschiede zwischen der Extensionskammer und der Flexionskammer aufgrund der einfahrenden bzw. ausfahrenden Kolbenstange. Der Kolben ist bevorzugt mit der Kolbenstange gekoppelt, die wiederum aus dem Gehäuse des Zylinders herausragt und linear beweglich ist.

Die Pumpe ist sowohl die Extensionskammer als auch die Flexionskammer befüllend betreibbar ausgebildet oder angeschlossen. Wenn die Pumpe nicht über die Drehrichtung umschaltbar ist, kann ein Umschaltventil in der strömungstechnischen Verbindung angeordnet sein, was allerdings den Steuerungsaufwand wieder erhöhen würde.

Die Rückschlagventile sind vorteilhafterweise als passive Ventile ausgebildet, beispielsweise federbelastete Kugelventile oder Klappenventile.

Die Stellventile sind vorteilhafterweise elektronisch gesteuert. Dazu können an der orthopädietechnischen Einrichtung wie Orthese, Prothese oder dem Patienten Sensoren angeordnet sein, die Zustandsgrößen über die orthopädietechnische Einrichtung oder den jeweiligen Bewegungszustand erfassen und einer Steuerungseinrichtung übermitteln, die mit einer Datenverarbeitungseinrichtung, beispielsweise einen Prozessor, ausgestattet ist. In dem Prozessor können Auswerteprogramme hinterlegt sein, um die Sensor-Rohdaten auszuwerten oder auf Grundlage der Sensorwerte Aktuatoren zu betätigen, um die Stellventile öffnen oder zu schließen. Die Stellventile können den Strömungsquerschnitt verringern oder vergrößern und gegebenenfalls vollständig verschließen, um die strömungstechnische Verbindung zwischen der Extensionskammer und der Flexionskammer zu sperren. Durch eine Verringerung des Strömungsquerschnittes wird der Strömungswiderstand vergrößert und eine sprechende Bewegung erschwert, bei einer Sperrung wird eine Bewegung unterbunden. Sind beide Stellventile vollständig geschlossen, ist der Aktuator gesperrt. Bei einer Anordnung des Aktuators an einem Gelenk ist damit eine Verlagerung des Gelenkes um eine Schwenkachse gesperrt.

Eine Weiterbildung der Erfindung sieht vor, dass mehrere Stellelemente mit der Pumpe strömungstechnisch gekoppelt sind. Als Stellelement werden insbesondere die mechanischen Komponenten mit dem Zylinder, der daraus herausragenden Kolbenstange und den Ventilen, gegebenenfalls mit Ausgleichsvolumen, angesehen. Das Stellelement kann mit einem separaten Gehäuse ausgebildet sein, das von dem Gehäuse der Pumpe getrennt ist und über Hydraulikleitungen mit der Pumpe gekoppelt ist. Dadurch ist es möglich, einen modularen Aufbau vorzusehen und eine einzelne Pumpe mit mehreren Stellelementen zu koppeln. So ist es möglich, eine einzelne Pumpe mit beispielsweise zwei Stellelementen hydraulisch zu verkoppeln, sodass jedes Stellelement einem anderen Gelenk zugeordnet werden kann. Ein erstes Stellelement kann beispielsweise als Aktuator und Widerstandseinrichtung für ein Orthesenkniegelenk oder Prothesenkniegelenk eingesetzt werden, während das zweite, ebenfalls mit der Pumpe gekoppelte Stellelement als Aktuator für ein Orthesenknöchelgelenk oder Prothesenknöchelgelenk eingesetzt werden kann.

Die Erfindung betrifft ebenfalls eine orthopädietechnische Einrichtung mit einem Hydraulikaktuator, wie er vorstehend beschrieben worden ist. Die orthopädietechnische Einrichtung ist insbesondere als eine Orthese oder Prothese ausgebildet, der Aktuator ist insbesondere als Widerstandseinrichtung und Antrieb für ein Gelenk innerhalb der orthopädietechnischen Einrichtung ausgebildet und geeignet.

Der Hydraulikaktuator ist als aktiver Aktuator oder als passiver Aktuator betreibbar ausgebildet und benötigt aufgrund der druckseitigen hydraulischen Ankopplung über ein Rückschlagventil keine mechatronische Steuerung für das Umschalten zwischen passiver Betriebsweise und aktiver Betriebsweise.

Das Stellelement kann zwischen einer Gelenkeinrichtung mit einem Oberteil und einem gelenkig daran befestigten Unterteil angeordnet sein. Eine Weiterbildung der Erfindung sieht vor, dass zwei an einer gemeinsamen Pumpe angeschlossene Stellelemente an unterschiedlichen Gelenkeinrichtungen der orthopädietechnischen Einrichtung angeordnet sind.

Nachfolgend werden Ausführungsbeispiele der Erfindung anhand der beigefügten Figuren näher erläutert. Es zeigen:
- Figur 1 -: eine schematische Darstellung einer Prothese mit Hydraulikaktuator;
- Figur 2 -: ein erstes hydraulisches Schaltbild für einen aktiven Betriebsmodus in 2 Richtungen;
- Figur 3-: ein hydraulisches Schaltbild für einen aktiven Betriebsmodus in einer Bewegungsrichtung;
- Figur 4 -: eine Variante der Figur 3;
- Figuren 5 bis 8 -: Strömungsverläufe für aktive und passive Betriebsweisen in einer Schaltung gemäß Figur 2; sowie
- Figur 9 -: eine Variante eine Hydraulikaktuators mit zwei Stellelementen.

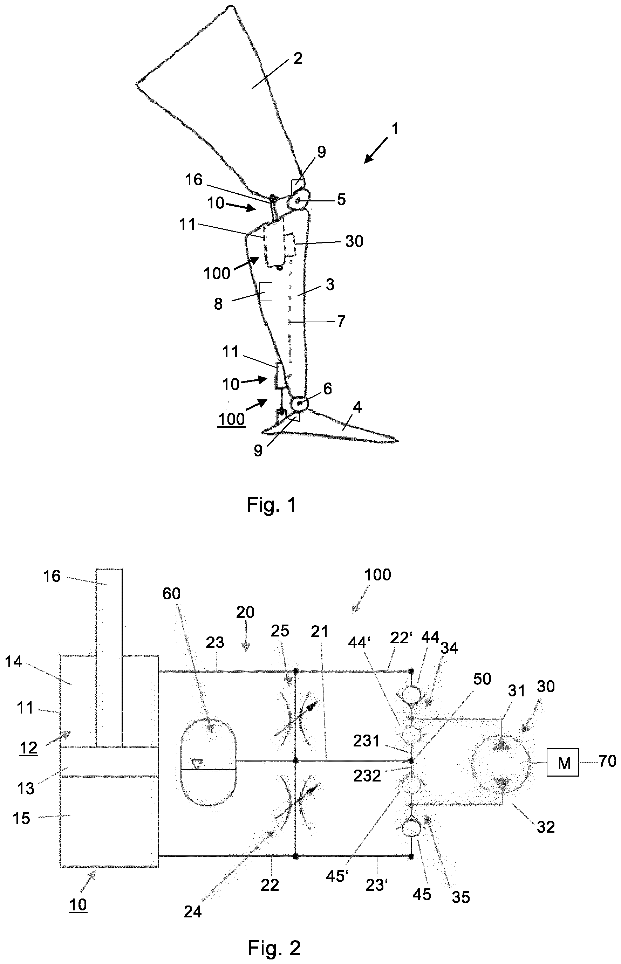

In der Figur 1 ist in einer schematischen Darstellung eine orthopädietechnische Einrichtung 1 in Gestalt einer Prothese dargestellt. Die orthopädietechnische Einrichtung 1 weist ein Oberteil 2 in Gestalt eines Prothesenschaftes auf, der über eine erste Gelenkeinrichtung 5 mit einem Unterteil 3 schwenkbar verbunden ist. An dem distalen Ende des Unterteils 3 ist über eine zweite Gelenkeinrichtung 6 ein Prothesenfuß 4 schwenkbar gelagert. Statt der Ausgestaltung der orthopädietechnischen Einrichtung 1 als eine Prothese, mit der ein nicht oder nicht mehr vorhandenes Bein ersetzt wird, kann die orthopädietechnische Einrichtung 1 auch als eine Orthese ausgebildet sein. Statt eines Oberschenkelschaftes 2, in dem ein Oberschenkelstumpf aufgenommen wird, um die Prothese an dem Patienten anzuordnen, kann das Oberteil 2 als eine Oberschenkelschiene oder Oberschenkelschale ausgebildet sein, die an dem Oberschenkel des Patienten angelegt wird. Das Unterteil 3 ist dann als eine Unterschenkelschiene oder Unterschenkelschale ausgebildet, die an dem Unterschenkel des Patienten festgelegt ist, beispielsweise über Riemen, Gurte oder Klettverschlüsse. An dem distalen Ende des Unterteils 3 kann statt eines Prothesenfußes 4 eine Fußauflage über die zweite Gelenkeinrichtung 6 schwenkbar an dem Unterteil 3 befestigt sein. Grundsätzlich ist es auch möglich, dass statt zweier Gelenkeinrichtungen 5, 6 die orthopädietechnische Einrichtung nur ein Gelenk überbrückt und beispielsweise als eine Unterschenkelprothese einen Unterschenkelschaft zur Aufnahme eines Unterschenkelstumpfes ausgebildet ist. An dem Unterschenkelschaft kann dann ein Prothesenfuß gelenkig befestigt sein; alternativ kann eine Orthese nur das Knöchelgelenk oder nur das Kniegelenk überbrücken, die jeweilige Gelenkeinrichtung ist dann auf der Höhe des jeweiligen natürlichen Gelenkes angeordnet. Weiterhin kann die orthopädietechnische Einrichtung auch an oberen Extremitäten angeordnet sein, beispielsweise als eine Orthese, die an einem Arm angelegt wird oder als Prothese einen Arm oder einen Teil eines Armes ersetzt. Ebenfalls kann die orthopädietechnische Einrichtung zu Anlage an dem Rumpf eines Nutzers ausgebildet sein. Bei einer Ausgestaltung der orthopädietechnischen Einrichtung 1 mit mehreren Gelenkeinrichtungen 5, 6 kann ein Unterteil für eine erste Gelenkeinrichtung 5 ein Oberteil für eine zweite Gelenkeinrichtung 6 bilden. Ein Oberteil ist dann diejenige Komponente, die proximal der Gelenkeinrichtung angeordnet ist, ein Unterteil ist diejenige Komponente, die distal der Gelenkeinrichtung angeordnet ist.

In dem dargestellten Ausführungsbeispiel der Figur 1 ist in dem Unterteil 3, das als Unterschenkelteil ausgebildet ist, ein Hydraulikaktuator 100 angeordnet, der ein Stellelement 10 mit einer daran angeordneten, motorisch angetriebenen Pumpe 30 aufweist. Der Hydraulikaktuator 100 weist ein Gehäuse 11 auf, das an dem Unterteil 3 ist festgelegt ist. Die Festlegung kann verschwenkbar erfolgen, um Relativbewegungen zwischen dem Gehäuse 11 und dem Unterteil 3 bei der Benutzung auszugleichen. Aus dem Gehäuse 11 des Stellelementes 10 ragt eine Kolbenstange 16 heraus, die an dem Oberteil 2 befestigt ist. Innerhalb des Hydraulikaktuators 100, der später näher erläutert werden wird, ist ein Zylinder mit einem darin verschieblich gelagerten Hydraulikkolben angeordnet, der über die Pumpe 30 aktiv verlagert werden kann. Durch die Verlagerung des Kolbens wird die Kolbenstange 16 ebenfalls verlagert, was zu einer Extension oder Flexion des Oberteils 2 relativ zu dem Unterteil 3 um die Schwenkachse der Gelenkeinrichtung 5 führt. Die Pumpe 30 kann in dem Gehäuse 11 des Hydraulikaktuators 100 integriert oder mit einem separaten Gehäuse ausgestattet und mit dem Hydraulikaktuator 100 über Leitungen gekoppelt sein.

In dem Ausführungsbeispiel gemäß Figur 1 ein zweiter Hydraulikaktuator 100 Bereich des Knöchelgelenkes angeordnet, der mit einem Stellelement 10 ausgebildet ist und über eine Hydraulikleitung 7 mit der Pumpe 30 des im Bereich des Kniegelenks angeordneten Hydraulikaktuators 100 gekoppelt ist. Wird die Pumpe 30 aktiviert, kann entweder der knieseitige Hydraulikaktuator 100 oder der knöchelseitige Hydraulikaktuator 100 mit Hydraulikfluid beaufschlagt werden. Grundsätzlich ist es auch möglich, beide Hydraulikaktuatoren 100 gleichzeitig mit Hydraulikfluid von der Pumpe 30 zu versehen.

Beide Hydraulikaktuatoren 100 können in einen aktiven Modus, bei dem die Pumpe 30 angetrieben ist, oder einem passiven Modus betrieben werden, wobei in dem passiven Modus der Hydraulikaktuator 100 als eine Widerstandseinrichtung, insbesondere als ein Hydraulikdämpfer arbeitet. An der orthopädietechnischen Einrichtung 1 können Sensoren 9 angeordnet sein, beispielsweise Kraftsensoren, Momentensensoren, Winkelsensoren, Lagesensoren, Beschleunigungssensoren, Gyroskope und/oder Inertial-Messeinheiten (IMU), die kabelgebunden oder kabellos mit einer Steuerungseinrichtung 8 verbunden sind. In der Steuerungseinrichtung 8 können Prozessoren oder andere Datenverarbeitungseinrichtungen untergebracht sein, über eine Batterie oder einen Akkumulator wird die Energieversorgung sichergestellt. Die Steuerungseinrichtung 8 verarbeitet die Sensorwerte und steuert Stellventile innerhalb des Hydraulikaktuators 100, um den Strömungswiderstand in Extensionsrichtung und/oder Flexionsrichtung an die jeweilige Belastung und/oder Gangsituation anzupassen. Grundsätzlich ist es auch möglich, dass das jeweilige Stellventil einmalig eingestellt und an den jeweiligen Nutzer angepasst wird.

In der Figur 2 ist ein hydraulisches Schaltbild des Hydraulikaktuators 100 dargestellt. Ein Stellelement 10 weist ein Gehäuse 11 mit einem darin ausgebildeten Zylinder 12 auf. Innerhalb des Zylinders 12 ist ein Hydraulikkolben 13 längsverschieblich angeordnet und unterteilt den Zylinder 12 in eine Flexionskammer 15 und eine Extensionskammer 14. An dem Kolben 13 ist eine Kolbenstange 16 angeordnet, die aus dem Gehäuse 11 hinausragt und mit einer Komponente der orthopädietechnischen Einrichtung 1, beispielsweise dem Oberteil 2 gekoppelt ist. Die andere Komponente der orthopädietechnischen Einrichtung 1 ist mit dem Gehäuse 11 gekoppelt. Bei einer Flexionsbewegung wird die Kolbenstange 16 in das Gehäuse 11 hinein bewegt und das Volumen der Flexionskammer 15 verringert. Gleichzeitig wird das Volumen der Extensionskammer 14 vergrößert, aufgrund des Volumens der Kolbenstange 16 ergibt sich eine Volumendifferenz zwischen den beiden Kammern 14, 15 bei einer Verlagerung. Die beiden Kammern 14, 15 sind über eine strömungstechnische Verbindung 20 miteinander gekoppelt, sodass Hydraulikfluid von der Flexionskammer 15 in die Extensionskammer 14 und umgekehrt fließen kann. Um den durch die Kolbenstange 16 hervorgerufenen Unterschied der Volumenveränderung auszugleichen, ist in der strömungstechnischen Verbindung 20 ein Ausgleichsvolumen 60 angeordnet, in das und aus dem Hydraulikfluid in die jeweilige Kammer 14, 15 geleitet werden kann.

In der Anordnung gemäß Figur 2 weist die strömungstechnische Verbindung 20 Anschlussleitungen 22, 23 auf, die von der jeweiligen Kammer 14, 15 abzweigen bzw. hinführen. Weiterhin sind in der strömungstechnischen Verbindung 20 zwei Stellventile 24, 25 angeordnet, durch die Hydraulikfluid durchgeleitet wird. Die Stellventile 24, 25 führen über eine Verbindungsleitung zu einer Zentralleitung 21, in die auch eine Leitung von dem Ausgleichsvolumen 60 mündet. In dem dargestellten Ausführungsbeispiel der Figur 2 ist das Stellventil 24 das Flexionsstellventil und das Stellventil 25 das Extensionsstellventil. Parallel zu der Verbindungsleitung sind parallele Zuführleitungen 231, 232 angeordnet, in denen Rückschlagventile 44, 44`, 45, 45' angeordnet sind. In den jeweiligen Zuführleitungen 231, 232 sind die Rückschlagventile als Rückschlagventilpaare 44, 44` bzw. 45, 45` angeordnet, wobei die Rückschlagventilpaare gleichgerichtet orientiert sind, sodass das Fluid aus der Zentralleitung 21 nur durch die Rückschlagventile 44, 44`, 45, 45` abfließen, jedoch nicht zurückströmen kann. Jeweils zwischen den Rückschlagventilpaaren 44, 44' bzw. 45, 45` ist eine Anschlussleitung 34, 35 angeordnet, die zu einem Einlass bzw. Auslass 31, 32 der Pumpe 30 führt. Je nach Antriebsrichtung oder Förderrichtung der Pumpe 30 werden die Anschlussleitungen 22, 23, 34, 35 zu saugseitigen oder druckseitigen Anschlussleitungen, ebenso kann ein Einlass zu einem Auslass werden und umgekehrt. Von den Rückschlagventilpaaren 44, 44` bzw. 45, 45' führen Rückführleitungen 22`, 23' zu den jeweiligen Kammern 14, 15, wobei die Rückführleitungen 22`, 23` in Anschlussleitungen 23, 22 münden können. Innerhalb dieser Leitungen, die von einem Rückschlagventil 44, 45 zur der jeweiligen Kammer 14, 15 führen, ist bevorzugt keine weitere strömungstechnische Beeinflussung vorgesehen, insbesondere kein Ventil oder eine Drossel. Das Stellelement 10 kann integriert die Pumpe 30 aufnehmen, ebenso können die Ventile innerhalb des Gehäuses 11 oder an dem Gehäuse 11 angeordnet sein, um eine möglichst kompakte Bauweise des Hydraulikaktuators 100 zu erreichen.

Durch die strömungstechnisch entgegengesetzt gerichtete Sperrung der Zuführleitungen 231, 232 wird eine Verbindung 50 zwischen der jeweils druckseitigen Anschlussleitung 34, 35, die von dem Rückschlagventil 44`, 45' zu der Pumpe 30 führt, zu einer saugseitigen Anschlussleitung 22, 23, durch die Hydraulikfluid aus dem Niederdruckbereich zu der Pumpe 30 gefördert wird, gesperrt. Durch die Schaltungsanordnung gemäß Figur 2 ist es möglich, den Hydraulikaktuator 100 aufgrund seines symmetrischen Aufbaus in beiden Betriebsrichtungen sowohl hinsichtlich der Flexion als auch der Extension zu betreiben. Ebenfalls ist es möglich, die Pumpe 30 deaktiv zu gestalten und aus dem Strömungskreislauf soweit zu entfernen, dass bei einem passiven Betrieb des Hydraulikaktuators 100 keine mit Druck beaufschlagten Hydraulikflüssigkeit an dem Pumpeneinlass anliegt.

Dadurch wird vermieden, dass bei einer Sperrung der Stellventile der Kolben 13 aufgrund von Dichtigkeitsproblemen oder einer bauartbedingten Leckage in der Pumpe 30 einsinkt Allein durch einen Drehrichtungsumkehr der Pumpe 30, beispielsweise durch Änderung der Drehrichtung des mit der Pumpe 30 verbundenen Motors 70, kann von einem aktiven Extensionsbetrieb auf einen aktiven Flexionsbetrieb umgeschaltet werden.

Durch die Anordnung der Pumpenanschlussleitungen 34, 35 innerhalb einer Rückschlagventil-Kaskade ist es möglich, bei einem aktiven Betriebsmodus die Pumpe 30 ohne Verstellung von Stellventilen in das System einzukoppeln. Mit Druck beaufschlagtes Hydraulikfluid wird aus einem Auslass der Pumpe 30 durch einen passives Rückschlagventil zu der jeweiligen Kammer des Hydraulikzylinders 12 geführt. Eine zusätzliche Ventilbetätigung zu Einkoppeln des Druckfluides ist nicht mehr nötig, wodurch sich der Steuerungsaufwand verringert und Bauraum eingespart wird. Das entsprechende Rückschlagventil 44, 45 öffnet sich erst, wenn der Druck von der Pumpe 30 größer als der Schließdruck des Rückschlagventils 44, 45 ist. Durch den Wegfall einer Ventilbetätigung wird ein zudem ruckhaftes Systemverhalten vermieden, welches auftritt, wenn nicht genau der Zeitpunkt des gleichen Druckes auf beiden Ventilseiten zum Öffnen des Ventils bei einem Stellventil erreicht wird. Darüber hinaus ist es möglich, dass bei einem Übergang von einem passiven Betriebsmodus zu einem aktiven Betriebsmodus die Pumpe 30 voreilend aktiviert werden kann, um eine gegenläufige Bewegung des Stellelementes 10 abzubremsen. Vorteilhafterweise wird für eine aktive Extension das Flexionsstellventil 24 geschlossen und das Extensionsstellventil 25 geöffnet, bei einer aktiven Flexion wird das Extensionsstellventil 25 geschlossen und das Flexionsstellventil 24 geöffnet.

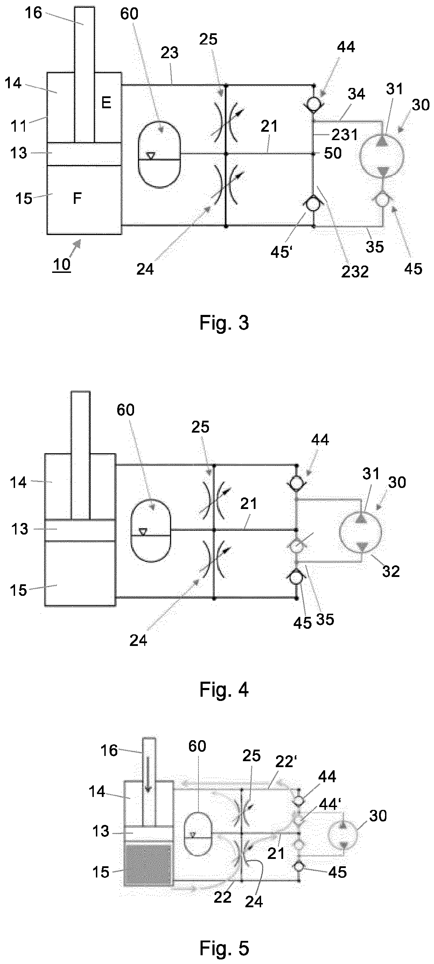

Wird in einer Ausführungsform nur eine Unterstützung in Extensionsrichtung gewünscht, also bei einem aktiven Betriebsmodus die Pumpe 30 nur in einer Richtung betrieben, ist ein mögliches hydraulisches Schaltbild hierfür in der Figur 3 gezeigt. Die Pumpe 30 saugt aus der Extensionskammer 14 Hydraulikfluid aus einer saugseitigen Anschlussleitung 23 durch das Extensionsstellventil 25 an, vorteilhafterweise ist das Flexionsstellventil 24 geschlossen. Durch die Zentralleitung 21 wird das Hydraulikfluid von der Verbindung 50 durch die Zuführleitung 231 saugseitige Anschlussleitung 34 zu dem Einlass 31 der Pumpe 31 geleitet. Erreicht und übersteigt der Pumpendruck den Druck, der durch das Hydraulikfluid aus der Flexionskammer 15 gegen das Rückschlagventil 45 aufgebracht wird, öffnet das passive Rückschlagventil 45 und Hydraulikfluid wird durch die druckseitige Anschlussleitung 35 in die Flexionskammer 15 gefördert. Ein Rückströmen in die Zentralleitung 21 wird durch das Rückschlagventil 45`, das parallel zu dem Rückschlagventil 45 in der druckseitigen Anschlussleitung 35 von der Pumpe 30 angeordnet ist, verhindert 24 kann ebenfalls kein Hydraulikfluid zu der Niederdruckseite bzw. zu der Zentralleitung 21 oder zu den Ausgleichsbehälter 60 gelangen, sodass bei entsprechendem Druckaufbau der Kolben 13 nach oben und die Kolbenstange 16 aus dem Gehäuse 11 ausgefahren wird, um eine Extensionsbewegung zu bewirken oder zu unterstützen. Zusätzlich zu dem Hydraulikfluid aus der Extensionskammer 14 wird Hydraulikfluid aus dem Ausgleichsvolumen 60 angesaugt und gefördert, um die Volumendifferenz durch die Kolbenstange 16 auszugleichen. Wird der Hydraulikaktuator 100 passiv betrieben, wird die Pumpe 30 nicht angetrieben und eine Beeinflussung der Extensionsbewegung oder Flexionsbewegung erfolgt durch einen veränderlichen Strömungsquerschnitt durch die einstellbaren Stellventile 24, 25. Bei einer Extension strömt das Fluid dann nicht mehr durch die Pumpe 30, sondern durch das parallel geschaltete Rückschlagventil 45' und die Zuführleitung 232 von der Zentralleitung 21. Umgekehrt strömt bei einer Flexion das Hydraulikfluid aus der Extensionskammer 14 durch das Flexionsstellventil 24 und das Rückschlagventil 44 in die Flexionskammer 15 und parallel dazu in das Ausgleichsvolumen 60.

Eine Variante der Verschaltung ist in der Figur 4 gezeigt, bei der ebenfalls nur eine aktive Unterstützung einer Extensionsbewegung vorgesehen ist. Die druckseitige Anschlussleitung 35, die von dem Auslass 32 der Pumpe 30 zu der Flexionskammer 15 führt, mündet zwischen zwei gleichgerichtet orientierten Rückschlagventile 45, 45`. Das eine Rückschlagventil 45' sperrt den Zufluss von der druckseitigen Anschlussleitung 35 zurück zu der Zentralleitung 21, das zweite Rückschlagventil 45 öffnet erst bei einer ausreichend großen Druckdifferenz zwischen der druckseitigen Anschlussleitung 35 und der Rückführleitung zu der Extensionskammer 14.

Auch hier ist vorteilhafterweise einem aktiven Betrieb das Flexionsstellventil 24 geschlossen, das Extensionsstellventil 25 ist geöffnet, sodass Druckfluid von der Flexionskammer 15 und dem Ausgleichsvolumen 60 durch die Zentralleitung 21 zu dem Einlass 31 strömen kann. Bei einer passiven Extensionsbewegung strömt das Hydraulikfluid nicht durch den Einlass 31, sondern durch die beiden Rückschlagventile 45, 45`, die bei einem passiven Betrieb auf der Niederdruckseite positioniert sind und ein Rückströmen in die Extensionskammer 14 ermöglichen. Gegebenenfalls kann das Flexionsstellventil 24 ein Durchströmen ermöglichen.

Das hydraulische Schaltbild der Figuren 3 und 4 ist für eine aktive Unterstützung einer Extensionsbewegung ausgelegt, bei einer alleinigen Flexionsunterstützung wäre der Anschluss der Pumpe 30 mit den Anschlussleitungen 34, 35 und die Anordnung der Rückschlagventile spiegelbildlich auf der anderen Seite der Zentralleitung 21 vorzunehmen

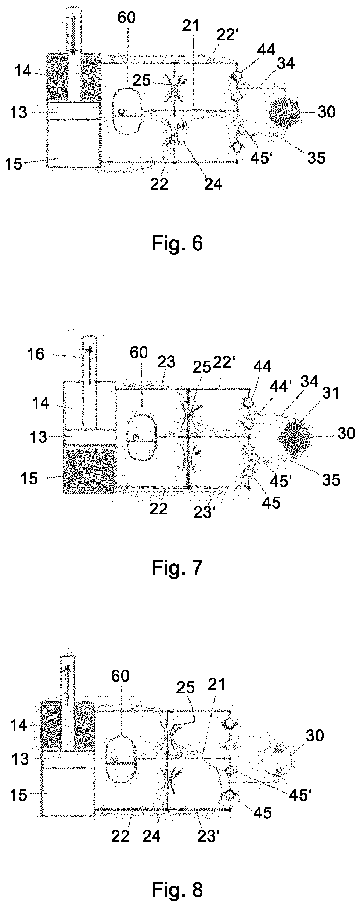

In den Figuren 5 bis 8 sind Schaltzustände und Strömungswege bei unterschiedlichen Betriebsmodi dargestellt. In der Figur 5 ist ein Schaltzustand für eine passive Flexion, in der Figur 6 für eine aktive Flexion mit angetriebener Pumpe 30, in der Figur 7 eine aktive Extension und in der Figur 8 eine passive Extension dargestellt. Die Verschaltung entspricht der Verschaltung in der Figur 2; aus Gründen der Übersichtlichkeit sind nicht alle Bezugszeichen eingezeichnet. Bei einer rein passiven Flexion, wie sie in der Figur 5 dargestellt ist, wird der Kolben 13 mit der Kolbenstange 16 nach unten bewegt, wie es durch den Pfeil angedeutet ist. Dadurch wird das Hydraulikfluid in der Flexionskammer 15 unter Druck gesetzt und strömt aus der Anschlussleitung 22 durch das Flexionsstellventil 24. Eine strömungstechnische Verbindung zu der Pumpe 30 ist durch das Rückschlagventil 45 gesperrt. Das Extensionsstellventil 25 kann ganz oder teilweise geöffnet sein. Das Hydraulikfluid strömt aus der Extensionskammer teilweise in das Ausgleichsvolumen 60 und durch die Zentralleitung 21 und das obere Rückschlagventilpaar 44, 44' in die Extensionskammer 14. Mit Druck beaufschlagtes Fluid aus der Extensionskammer 15 wird nicht zu einem Einlass der Pumpe 30 geleitet.

Wird hingegen eine aktive Unterstützung gewünscht, wird bevorzugt das Extensionsstellventil 25 geschlossen und das Flexionsstellventil 24 geöffnet. Hydraulikfluid wird aus der Extensionskammer 14 angesaugt und befindet sich somit in einem Niederdruckbereich. Durch die Zentralleitung 21 und das Rückschlagventil 42' wird das Fluid durch eine saugseitige Anschlussleitung 35 angesaugt. In der Pumpe 30 erfolgt eine Druckerhöhung. Das unter Druck gesetzte Fluid wird durch eine druckseitige Anschlussleitung 34 und ein Rückschlagventil 45, das ein Rückströmen zu der Pumpe 30 sperrt, durch eine Rückführleitung 22 zu der Flexionskammer 15 gepumpt. Der Kolben 13 wird nach unten gedrückt, die Kolbenstange 16 wird eingefahren und eine Flexion aktiv bewirkt oder unterstützt. Ein Teilvolumenstrom strömt aus der Extensionskammer 14 das Ausgleichsvolumen 60.

Bei einer aktiven Extension, wie sie in der Figur 7 gezeigt ist, strömt Fluid aus der Flexionskammer 15 durch eine saugseitige Anschlussleitung 23 durch ein geöffnetes Extensionsstellventil 25. Zusätzlich benötigtes Hydraulikfluid strömt aus dem Ausgleichsvolumen 60 mit in die Zentralleitung 21 und von dort durch das Rückschlagventil 44' zu dem Einlass 31 der Pumpe 30. Ein zweites, gleichgerichtetes Rückschlagventil 44 sperrt einen direkten Zufluss von der Extensionskammer 14 zu einem Pumpeneinlass 31. Von der angetriebene Pumpe 30 strömt Hydraulikfluid durch eine druckseitige Anschlussleitung 35 durch ein Rückschlagventil 45 durch die Rückführleitung 23' und die als Anschlussleitung 22 bei einer umgekehrten Bewegung fungierende Leitung in die Flexionskammer 15 bewirkt ein Herausfahren der Kolbenstange 16 damit eine Extensionsbewegung.

In der passiven Schaltung gemäß Figur 8 bleibt die Pumpe 30 deaktiviert, Hydraulikfluid strömt aus der Extensionskammer 14, dem Extensionsstellventil 25, dem Ausgleichsvolumen 60 durch das Rückschlagventilpaar 45, 45` und gegebenenfalls durch ein geöffnetes oder teilweise geöffnetes Flexionsstellventil 24 in die Flexionskammer 15.

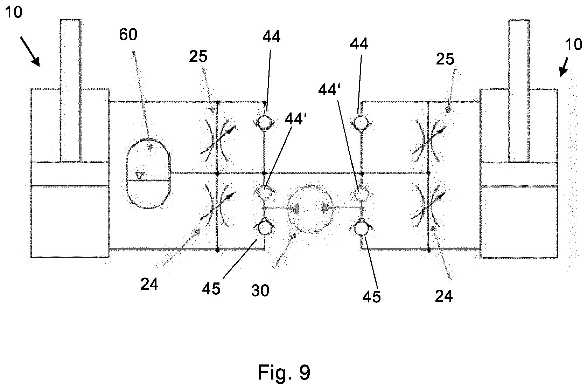

In der Figur 9 ist eine weitere Variante des Hydraulikaktuators 100 dargestellt, bei der zwei Stellelemente 10 miteinander gekoppelt sind. Der grundsätzliche Aufbau der Verschaltung entspricht im Wesentlichen der Verschaltung, die in der Figur 2 dargestellt ist. Daher sind nicht alle Bezugszeichen in der Figur 9 eingezeichnet. Abweichend zu der Figur 2 ist die motorisch angetriebene Pumpe 30 so verschaltet, dass beide Stellelemente 10 in zumindest eine Arbeitsrichtung oder Bewegungsrichtung angetrieben werden können. Ist ein Stellelement 10 beispielsweise an einem künstlichen Kniegelenk und das andere Stellelement 10 an einem künstlichen Knöchelgelenk angeordnet, kann beispielsweise eine Plantarflexion und eine Knieextension gleichzeitig durch die Pumpe 30 verursacht werden. Alternativ dazu kann die Verschaltung dergestalt ausgebildet sein, dass eine Knieflexion und eine Dorsalflexion oder eine andere Verknüpfung der jeweiligen Flexionsbewegung oder Extensionsbewegung erfolgt. Auch bei der Ausgestaltung gemäß Figur 9 münden die Anschlussleitungen zu einem Einlass bzw. Auslass der Pumpe 30 zwischen zwei gleichgerichtete Rückschlagventile 44`, 45. Von dort wird gepumptes Hydraulikfluid über die Rückführleitungen bzw. Anschlussleitungen zu den jeweiligen Kammern der Stellelemente 10 geleitet. Es ist nur ein gemeinsames Ausgleichsvolumen 60 vorhanden, das an der Zentralleitung angeschlossen ist. Beide Stellelemente 10 weisen Flexionsstellventile 24 und Extensionsstellventile 25 auf, über die es möglich ist, unterschiedliche Widerstände der jeweiligen Stellelemente 10 bei einem passiven Betrieb einzustellen oder durch die Veränderung der Strömungswiderstände unterschiedliche Verstellwege bei den jeweiligen Stellelementen 10 zu erreichen. Auch mit zwei Stellelementen 10 ist es möglich, die Pumpe 30 bei einem aktiven oder passiven Betrieb von der Hochdruckseite zu entkoppeln und nur noch Hydraulikfluid aus dem Niederdruckbereich der Pumpe 30 zuzuführen. Dies wird durch die dargestellte Verschaltung und Anordnung der Rückschlagventile 44, 44`, 45 und die Anordnung der Stellventile 24, 25 parallel dazu erreicht.

## Patentansprüche

1. Hydraulikaktuator für Orthesen oder Prothesen (1) mit einem Stellelement (10) mit einem Gehäuse (11), in dem ein Zylinder (12) angeordnet ist, in dem ein Kolben (13) beweglich gelagert ist und den Zylinder (12) in eine Flexionskammer (15) und eine Extensionskammer (14) unterteilt, zwischen der Flexionskammer (15) und der Extensionskammer (14) ist eine strömungstechnische Verbindung (20) angeordnet, in der je ein Stellventil (24, 25) zur Beeinflussung der Extensionsbewegung bzw. Flexionsbewegung angeordnet ist, wobei eine motorisch angetriebene Pumpe (30) in der strömungstechnischen Verbindung (20) angeordnet ist, **dadurch gekennzeichnet, dass** das Hydraulikfluid von der einen Kammer (14; 15) durch zumindest ein Stellventil (24; 25) in einer saugseitigen Anschlussleitung (22; 23) zu der Pumpe (30) geleitet wird und ein Rückschlagventil (44; 45) in einer druckseitigen Anschlussleitung (34, 35) von der Pumpe (30) zu der anderen Kammer (15; 14) angeordnet ist, das ein Rückströmen des gepumpten Mediums aus dieser Kammer (15; 14) zu der Pumpe (30) entgegen der Förderrichtung der Pumpe (30) sperrt, wobei die druckseitige Anschlussleitung (34, 35), mit der die Pumpe (30) an die strömungstechnische Verbindung (20) angeschlossen ist, zwischen zwei gleichgerichtet wirkenden, in Reihe angeordneten Rückschlagventilen (44, 44'; 45, 45') mündet.

2. Hydraulikaktuator nach Anspruch 1, **dadurch gekennzeichnet, dass** zwischen der Pumpe (30) und der von der Pumpe (30) mit Druck beaufschlagten Kammer nur ein Rückschlagventil (44; 45) angeordnet ist.

3. Hydraulikaktuator nach einem der voranstehenden Ansprüche, **dadurch gekennzeichnet, dass** ein Rückschlagventil (44', 45') eine Verbindung (50) zwischen der druckseitigen Anschlussleitung (34; 35) und einer saugseitigen Anschlussleitung (22; 23) sperrt.

4. Hydraulikaktuator nach einem der voranstehenden Ansprüche, **dadurch gekennzeichnet, dass** in der strömungstechnischen Verbindung (20) eine Zentralleitung (21) ausgebildet ist, in die die saugseitigen Anschlussleitungen (22, 23) von der Flexionskammer (15) und der Extensionskammer (14) münden und von der Zuführleitungen (231, 232) zu zumindest einem saugseitigen Einlass (31; 32) zu der Pumpe (30) führen.

5. Hydraulikaktuator nach Anspruch 4, **dadurch gekennzeichnet, dass** in den saugseitigen Anschlussleitungen (22, 23) jeweils ein Stellventil (24, 25) angeordnet ist.

6. Hydraulikaktuator nach einem der voranstehenden Ansprüche, **dadurch gekennzeichnet, dass** die Pumpe (30) zwei Druckanschlüsse aufweist, die über je eine druckseitige Anschlussleitung (34, 35) an die strömungstechnische Verbindung (20) angeschlossen sind, wobei beide druckseitigen Anschlussleitungen (34, 35) zwischen zwei gleichgerichtet wirkenden, in Reihe angeordneten Rückschlagventilen (44, 44'; 45, 45'), die als Rückschlagventilpaare ausgebildet sind, münden, wobei die Rückschlagventilpaare entgegengesetzt gerichtete Durchflussrichtungen aufweisen.

7. Hydraulikaktuator nach einem der Ansprüche 4 bis 6, **dadurch gekennzeichnet, dass** die Zentralleitung (21) zu Rückführleitungen (22', 23') zu der Extensionskammer (14) und Flexionskammer (15) führt und in der Rückführleitung (22', 23') zumindest ein Rückschlagventil (44, 44'; 45, 45') angeordnet ist, das ein Rückströmen in die Zentralleitung (21) verhindert.

8. Hydraulikaktuator nach einem der voranstehenden Ansprüche, **dadurch gekennzeichnet, dass** die druckseitige Anschlussleitung (34, 35) zu einer Rückführleitung (22', 23') zu der Extensionskammer (14) oder Flexionskammer (15) führt.

9. Hydraulikaktuator nach einem der voranstehenden Ansprüche, **dadurch gekennzeichnet, dass** ein Ausgleichsvolumen (60) an der strömungstechnischen Verbindung (20) angeschlossen ist.

10. Hydraulikaktuator nach einem der voranstehenden Ansprüche, **dadurch gekennzeichnet, dass** der Kolben (13) mit einer Kolbenstange (16) gekoppelt ist, die aus dem Gehäuse (11) herausragt und linear beweglich gelagert ist.

11. Hydraulikaktuator nach einem der voranstehenden Ansprüche, **dadurch gekennzeichnet, dass** die Pumpe (30) sowohl die Extensionskammer (14) als auch die Flexionskammer (15) befüllend betreibbar ausgebildet oder angeschlossen ist.

12. Hydraulikaktuator nach einem der voranstehenden Ansprüche, **dadurch gekennzeichnet, dass** die Rückschlagventile (44, 44`; 45, 45') als passive Ventile ausgebildet sind.

13. Hydraulikaktuator nach einem der voranstehenden Ansprüche, **dadurch gekennzeichnet, dass** die Stellventile (24, 25) elektronisch gesteuert sind.

14. Hydraulikaktuator nach einem der voranstehenden Ansprüche, **dadurch gekennzeichnet, dass** mehrere Stellelemente (10) mit der Pumpe (30) strömungstechnisch gekoppelt sind.

15. Orthopädietechnische Einrichtung (1) mit einem Hydraulikaktuator (100) nach einem der voranstehenden Ansprüche.

16. Orthopädietechnische Einrichtung nach Anspruch 15, **dadurch gekennzeichnet, dass** der Hydraulikaktuator (100) als aktiver Aktuator oder als passiver Aktuator (100) betreibbar ausgebildet ist.

17. Orthopädietechnische Einrichtung nach Anspruch 15 oder 16, **dadurch gekennzeichnet, dass** das Stellelement (10) zwischen einer Gelenkeinrichtung (5, 6) mit einem Oberteil (2; 3) und einem gelenkig daran befestigten Unterteil (3; 4)angeordnet ist.

18. Orthopädietechnische Einrichtung nach einem der Ansprüche 15 bis 17, **dadurch gekennzeichnet, dass** zwei an einer gemeinsamen Pumpe (30) angeschlossene Stellelemente (10) an unterschiedlichen Gelenkeinrichtungen (5, 6) der orthopädietechnischen Einrichtung (1) angeordnet sind.

## Claims

1. A hydraulic actuator for orthoses or prostheses (1), comprising a control element (10) with a housing (11) in which a cylinder (12) is arranged, in which a piston (13) is mounted movably and divides the cylinder (12) into a flexion chamber (15) and an extension chamber (14), wherein a fluidic connection (20) is arranged between the flexion chamber (15) and the extension chamber (14), in which fluidic connection (20) a control valve (24, 25) is arranged for influencing the extension movement and flexion movement respectively, wherein a motor-driven pump (30) is arranged in the fluidic connection (20), **characterized in that** the hydraulic fluid is conveyed from one chamber (14; 15) to the pump (30) through at least one control valve (24; 25) in a suction-side connection line (22; 23), and a check valve (44; 45) is arranged in a pressure-side connection line (34, 35) from the pump (30) to the other chamber (15; 14) and blocks a backflow of the pumped medium from this chamber (15; 14) to the pump (30) counter to the delivery direction of the pump (30), wherein the pressure-side connection line (34, 35), with which the pump (30) is connected to the fluidic connection (20), opens out between two check valves (44, 44'; 45, 45') which act in the same direction and are arranged in series.

2. The hydraulic actuator as claimed in claim 1, **characterized in that** only one check valve (44; 45) is arranged between the pump (30) and the chamber pressurized by the pump (30).

3. The hydraulic actuator as claimed in one of the preceding claims, **characterized in that** a check valve (44', 45') blocks a connection (50) between the pressure-side connection line (34; 35) and a suction-side connection line (22; 23).

4. The hydraulic actuator as claimed in one of the preceding claims, **characterized in that** a central line (21) is formed in the fluidic connection (20), into which central line (21) the suction-side connection lines (22, 23) from the flexion chamber (15) and the extension chamber (14) open, and from which supply lines (231, 232) lead to at least one suction-side inlet (31; 32) to the pump (30).

5. The hydraulic actuator as claimed in claim 4, **characterized in that** a control valve (24, 25) is arranged in each of the suction-side connection lines (22, 23).

6. The hydraulic actuator as claimed in one of the preceding claims, **characterized in that** the pump (30) has two pressure connections which are each connected to the fluidic connection (20) via a respective pressure-side connection line (34, 35), wherein both pressure-side connection lines (34, 35) open out between two check valves (44, 44'; 45, 45') which are designed as pairs of check valves and which act in the same direction and are arranged in series, wherein the pairs of check valves have oppositely directed flow directions.

7. The hydraulic actuator as claimed in one of claims 5 through 7, **characterized in that** the central line (21) leads to return lines (22', 23') to the extension chamber (14) and flexion chamber (15), and in the return line (22', 23') at least one check valve (44, 44'; 45, 45') is arranged which prevents a backflow into the central line (21).

8. The hydraulic actuator as claimed in one of the preceding claims, **characterized in that** the pressure-side connection line (34, 35) leads to a return line (22', 23') to the extension chamber (14) or flexion chamber (15).

9. The hydraulic actuator as claimed in one of the preceding claims, **characterized in that** a compensation volume (60) is connected to the fluidic connection (20).

10. The hydraulic actuator as claimed in one of the preceding claims, **characterized in that** the piston (13) is coupled to a piston rod (16) which protrudes from the housing (11) and which is mounted so as to be linearly movable.

11. The hydraulic actuator as claimed in one of the preceding claims, **characterized in that** the pump (30) is designed or connected so as to be able to be operate to fill both the extension chamber (14) and the flexion chamber (15).

12. The hydraulic actuator as claimed in one of the preceding claims, **characterized in that** the check valves (44, 44'; 45, 45') are designed as passive valves.

13. The hydraulic actuator as claimed in one of the preceding claims, **characterized in that** the control valves (24, 25) are electronically controlled.

14. The hydraulic actuator as claimed in one of the preceding claims, **characterized in that** a plurality of control elements (10) are fluidically coupled to the pump (30).

15. An orthopedic device (1) with a hydraulic actuator (100) as claimed in one of the preceding claims.

16. The orthopedic device as claimed in claim 15, **characterized in that** the hydraulic actuator (100) is designed to be able to be operated as an active actuator or as a passive actuator (100).

17. The orthopedic device as claimed in claim 15 or 16, **characterized in that** the control element (10) is arranged between a joint device (5, 6) with an upper part (2; 3) and with a lower part (3; 4) attached to the latter in an articulated manner.

18. The orthopedic device as claimed in one of claims 15 through 17, **characterized in that** two control elements (10) connected to a common pump (30) are arranged on different joint devices (5, 6) of the orthopedic device (1).

## Revendications

1. Actionneur hydraulique pour orthèses ou prothèses (1), comprenant un élément de réglage (10) muni d'un boîtier (11) dans lequel est disposé un cylindre (12) dans lequel un piston (13) est monté de façon mobile et qui subdivise le cylindre (12) en une chambre de flexion (15) et en une chambre d'extension (14), une communication fluidique (20) étant disposée entre la chambre de flexion (15) et la chambre d'extension (14), communication dans laquelle est disposée une vanne de réglage (24, 25) respective destinée à influencer respectivement le mouvement d'extension et le mouvement de flexion, une pompe (30) entraînée par voie motrice étant disposée dans la communication fluidique (20),
**caractérisé en ce que** le fluide hydraulique est amené de l'une des chambres (14 ; 15) vers la pompe (30) par au moins une vanne de réglage (24 ; 25) située dans une conduite de raccordement côté aspiration (22 ; 23), et un clapet anti-retour (44 ; 45) est disposé dans une conduite de raccordement côté refoulement (34, 35) de la pompe (30) vers l'autre chambre (15 ; 14), clapet qui bloque un reflux du fluide pompé de cette chambre (15 ; 14) vers la pompe (30) en sens opposé à la direction de refoulement de la pompe (30), et la conduite de raccordement côté refoulement (34, 35) par laquelle la pompe est raccordée à la communication fluidique (20) débouche entre deux clapets anti-retour (44, 44' ; 45, 45') disposés en série et agissant dans le même sens.

2. Actionneur hydraulique selon la revendication 1,
**caractérisé en ce qu'**un seul clapet anti-retour (44 ; 45) est disposé entre la pompe (30) et la chambre mise sous pression par la pompe (30).

3. Actionneur hydraulique selon l'une des revendications précédentes,
**caractérisé en ce qu'**un clapet anti-retour (44', 45') bloque une communication (50) entre la conduite de raccordement côté refoulement (34 ; 35) et une conduite de raccordement côté aspiration (22 ; 23).

4. Actionneur hydraulique selon l'une des revendications précédentes,
**caractérisé en ce qu'**une conduite centrale (21) est formée dans la communication fluidique (20), dans laquelle débouchent les conduites de raccordement côté aspiration (22, 23) de la chambre de flexion (15) et de la chambre d'extension (14) et depuis laquelle des conduites d'alimentation (231, 232) mènent à au moins une entrée côté aspiration (31 ; 32) vers la pompe (30).

5. Actionneur hydraulique selon la revendication 4,
**caractérisé en ce qu'**une vanne de réglage (24, 25) respective est disposée dans chacune des conduites de raccordement côté aspiration (22, 23).

6. Actionneur hydraulique selon l'une des revendications précédentes,
**caractérisé en ce que** la pompe (30) présente deux raccords de refoulement qui sont raccordés à la communication fluidique (20) chacun par une conduite de raccordement côté refoulement (34, 35), les deux conduites de raccordement côté refoulement (34, 35) débouchant entre deux clapets anti-retour (44, 44' ; 45, 45') disposés en série et agissant dans le même sens, qui sont conçus comme des paires de clapets anti-retour, les paires de clapets anti-retour présentant des sens d'écoulement opposés.

7. Actionneur hydraulique selon l'une des revendications 4 à 6,
**caractérisé en ce que** la conduite centrale (21) mène à des conduites de retour (22', 23') vers la chambre d'extension (14) et la chambre de flexion (15), et au moins un clapet anti-retour (44, 44' ; 45, 45') est disposé dans la conduite de retour (22', 23'), lequel empêche un reflux dans la conduite centrale (21).

8. Actionneur hydraulique selon l'une des revendications précédentes,
**caractérisé en ce que** la conduite de raccordement côté refoulement (34, 35) mène à une conduite de retour (22', 23') vers la chambre d'extension (14) ou la chambre de flexion (15).

9. Actionneur hydraulique selon l'une des revendications précédentes,
**caractérisé en ce qu'**un volume de compensation (60) est raccordé à la communication fluidique (20).

10. Actionneur hydraulique selon l'une des revendications précédentes,
**caractérisé en ce que** le piston (13) est couplé à une tige de piston (16) qui fait saillie hors du boîtier (11) et qui est montée de façon mobile linéairement.

11. Actionneur hydraulique selon l'une des revendications précédentes,
**caractérisé en ce que** la pompe (30) est conçue ou raccordée de manière à pouvoir remplir aussi bien la chambre d'extension (14) que la chambre de flexion (15).

12. Actionneur hydraulique selon l'une des revendications précédentes,
**caractérisé en ce que** les clapets anti-retour (44, 44' ; 45, 45') sont conçus comme des vannes passives.

13. Actionneur hydraulique selon l'une des revendications précédentes,
**caractérisé en ce que** les vannes de réglage (24, 25) sont commandées par voie électronique.

14. Actionneur hydraulique selon l'une des revendications précédentes,
**caractérisé en ce que** plusieurs éléments de réglage (10) sont couplés fluidiquement à la pompe (30).

15. Dispositif orthopédique (1) comprenant un actionneur hydraulique (100) selon l'une des revendications précédentes.

16. Dispositif orthopédique selon la revendication 15,
**caractérisé en ce que** l'actionneur hydraulique (100) est conçu pour fonctionner comme un actionneur actif ou comme un actionneur passif (100).

17. Dispositif orthopédique selon la revendication 15 ou 16,
**caractérisé en ce que** l'élément de réglage (10) est disposé entre un dispositif d'articulation (5, 6) ayant une partie supérieure (2 ; 3) et une partie inférieure (3 ; 4) qui y est fixée de manière articulée.

18. Dispositif orthopédique selon l'une des revendications 15 à 17,
**caractérisé en ce que** deux éléments de réglage (10) raccordés à une pompe commune (30) sont disposés sur différents dispositifs d'articulation (5, 6) du dispositif orthopédique (1).
